# EUROPEAN PATENT APPLICATION

(11) **EP 0 791 339 A1**
(43) Date of publication of application: **27.08.1997**
(21) Application number: 97301207.3
(22) Date of filing: 25.02.1997
(51) Int. Cl.: A61B 19/00

(54) **Stand apparatus for microsurgery aid**

(30) Priority: 26.02.1996 JP 38472/96
(71) Applicant: MITAKA KOHKI CO., LTD., Mitaka-shi Tokyo 181 (JP)
(72) Inventor: Nakamura, Katsushige, Hachioji-shi, Tokyo 193 (JP)
(74) Representative: Skone James, Robert Edmund

(57) **Abstract**

The distal end (15) of a holder frame (14) bifurcates to have a fork-like shape, and a microscope (W₁) is supported between the bifurcated distal end (15). Auxiliary devices are mounted to the side surfaces of the bifurcated distal end (15). The auxiliary devices are not mounted on the microscope but on the holder frame (14) and so will not rotate if the microscope (W₁) is rotated. Therefore, the rotated microscope (W₁) does not affect the good balance kept by the stand apparatus (A).

## Description

The present invention relates to a stand apparatus for a microsurgery aid which allows heavy devices such as an operating microscope and its auxiliary devices to be located at a desired spatial position during microsurgery.

The fields of brain surgery and cardiac surgery have an operating method called microsurgery in which microscopes are used to view and inspect an affected part during the operations. For such microsurgery, there have been proposed some microsurgery aids which permit a stand apparatus to hold at a desired spatial position such heavy devices as an operating microscope and its auxiliary devices. A typical type of the stand apparatus used for the microsurgery aid generally has a structure utilizing parallel linkage and is provided with a counterweight for countervailing the microscope and the like to be held by the stand apparatus.

A microscope used in a microsurgery aid is rotatably supported at a holding frame of a stand apparatus. The microscope often has various auxiliary devices mounted thereto including a side microscope for an assistant doctor and a video camera, so that every time the microscope is rotated, the auxiliary devices will be rotated together.

In the prior art, even if the microscope and its auxiliary devices are supported by the stand apparatus in a well-balanced state when they are prepared, rotation of the microscope in service will cause a simultaneous rotation of the heavy auxiliary devices, and which may make the whole weight balance preliminary adjusted between the microscope and the like and auxiliary devices lost. If rotated in accordance with the rotation of the microscope, the auxiliary devices may hit a neighbour assistant, and it is also not preferable for safety reasons.

In accordance with the present invention, a stand apparatus for a microsurgery aid comprises a holder frame moveable to any optional spatial position and being adapted to support a microscope and one or more auxiliary devices at the distal end, and is characterised in that the distal end of said holder frame bifurcates to have a fork-like shape between which bifurcated distal end a microscope is supported in use, at least one side of the bifurcated distal end being adapted to support an auxiliary device.

The present invention has been made while attention was being paid to the prior art, and provides a stand for a microsurgery aid capable of immobilizing the auxiliary device(s) when the microscope is rotated and maintaining a good optical relationship with the auxiliary devices.

In the invention, the auxiliary device(s) are mounted not at the microscope but at the holder frame and so will not rotate if the microscope is rotated. Therefore, the rotated microscope does not affect the good balance kept by the stand apparatus.

Preferably, the distal end of said holder frame includes at least one optical output to which a microscope can be rotatably supported, the auxiliary device being optically connected in use to the optical output.

The microscope rotatably supported at the distal ends of the holder frame about the optical output defines at the pivot centre of the microscope a passage for light flux and allows an optical connection between the microscope and the auxiliary devices easily.

Typically, the side surfaces of the bifurcated distal end are adapted to support auxiliary device(s).

Preferably, the stand apparatus further comprises reflection means for passing light from a microscope mounted to the stand into at least one optical output, the reflection means including a prism or mirror which is coupled to a microscope, in use, so as to rotate relative to the optical output by half the rotated angle of the microscope.

Prisms or mirrors rotating just by half the rotated angle of the microscope do not allow an image available from the optical output to rotate if the microscope is rotated.

In some cases, an image rotator is provided in the optical output and rotates just by half the rotated angle of the microscope, and further manually rotatable just by optional degrees.

This allows an image available from the optical output optionally to rotate when an image rotator is manually rotated. The image rotator is designed to rotate only half the rotated angle of the microscope normally, so that rotation of the microscope does not rotate the image.

The present invention is not limited to the above description, and its other objects, advantages, features, and applications will become more apparent from the following description in conjunction with the accompanying drawings. It should be understood that changes and modifications are possible without departing from the spirit and scope of the invention.
Fig. 1 is a perspective view of a medical stand apparatus showing one embodiment of the present invention;
Fig. 2 is a side view of the medical stand apparatus;
Fig. 3 is a side view taken from the direction indicated by an arrow DA in Fig. 2;
Fig. 4 is an enlarged sectional side view illustrating a holding frame structure and an internal optical mechanism of a microscope, taken from the direction indicated by an arrow DB in Fig. 1;
Fig. 5 is a sectional view taken along the line SA-SA indicated by an arrow in Fig. 4;
Fig. 6 is a plan view taken from the direction indicated by an arrow DC in Fig. 4; and
Fig. 7 is a side view illustrating an ocular and a counterweight.

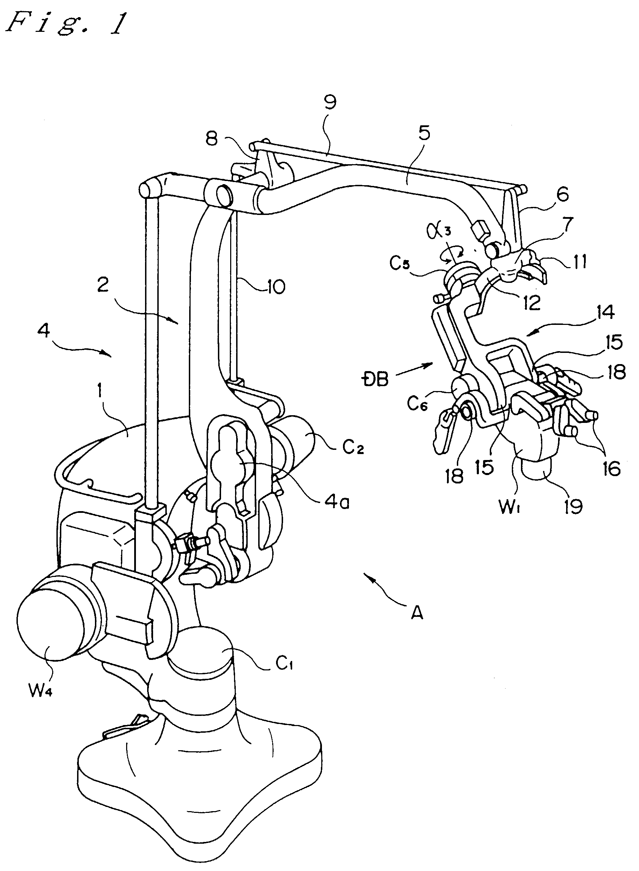

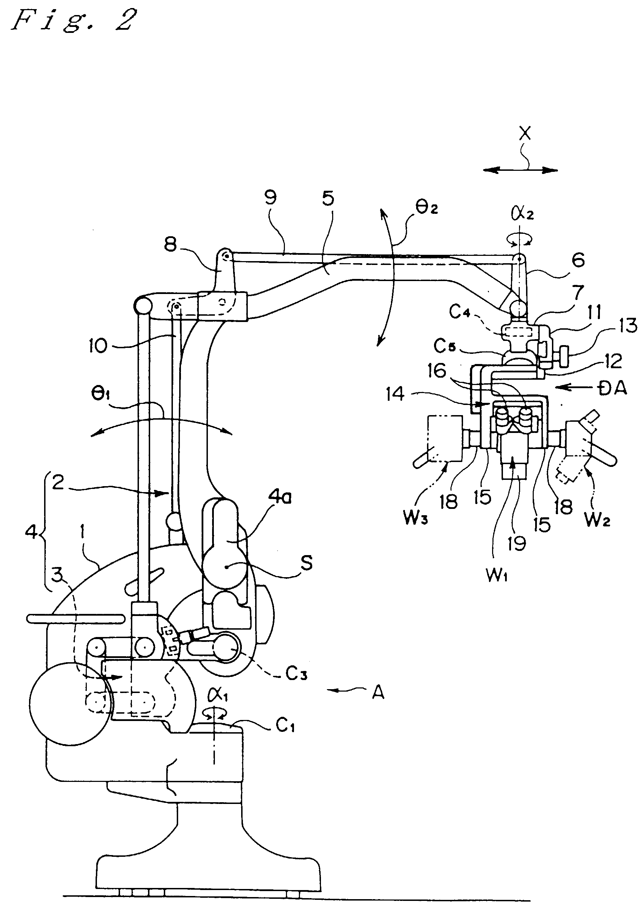

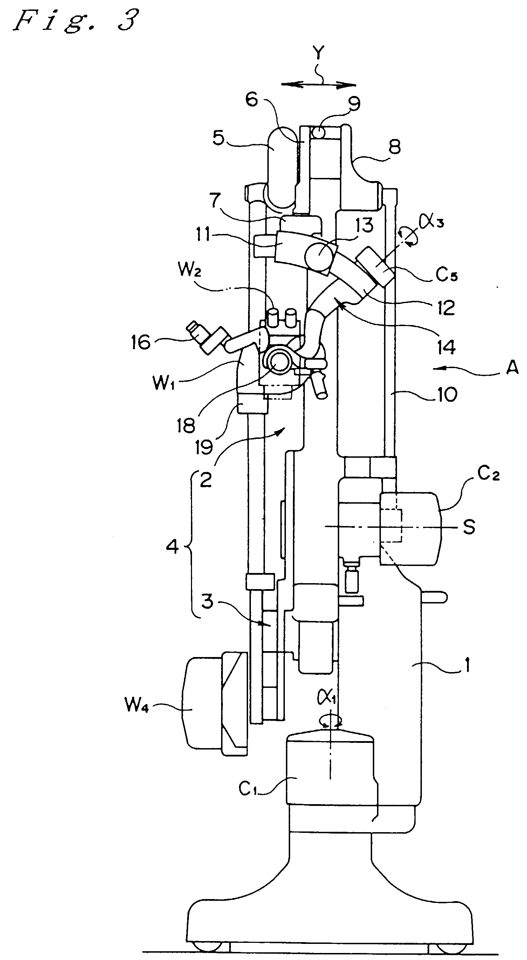

A preferred embodiment of the present invention will be described with reference to the accompanying drawings. First of all, description is given to the whole stand apparatus A which is used in a microsurgery aid of the embodiment. Fig. 1 is a perspective view illustrating the whole apparatus, Fig. 2 is a side view of the apparatus, and Fig. 3 is a side view taken from the direction indicated by an arrow DA in Fig. 2. Figs. 2 and 3 illustrate the same conditions of the apparatus, and Fig. 1 is modified so that Figs. 2 and 3 and the orientation of a microscope W₁ and the like are readily understood.

The stand apparatus A according to the embodiment is designed to be installed on a floor of an operating room, and a mount 1 is rotatable about an axis α ₁. The rotation of the mount 1 about the axis α ₁ is locked by an electromagnetic clutch C₁. The electromagnetic clutch C₁ is normally closed by a heavy-duty spring not shown. The clutch is firmly locked by the spring when not energized, and unlocked only when energized for rotation or swivel. Thus, when an unforeseen accident, for example power failure happens, the clutch is automatically locked and safety is secured. The following "electromagnetic clutches" have a structure identical to the above discussed clutch.

The mount 1 has a horizontal center pivot S supporting a middle part 4a of a retaining link mechanism 4 which comprises a first parallel link assembly 2 and a second parallel link assembly 3. The whole retaining link mechanism 4 is vertically moveable relative to the pivot S of the mount 1, and also vertically adjustable in balance relative to the pivot S of the retaining link mechanism 4. The pivot S is provided with an electromagnetic clutch C₂ (see Fig. 3) which contributes to lock displacement θ ₁ (see Fig. 2) in the crosswise directions of the retaining link mechanism 4. The upper part of the retaining link mechanism 4 has a jib arm 5 integrally formed therewith, and the distal end of the jib arm 5 is provided with a vertical front arm 6. A lower end 7 of the front arm 6 holds the microscope W_{1.} A detailed description of a holding structure will be given later. The jib arm 5 is subject to vertical displacement θ₂ (see Fig.2). The vertical displacement θ₂ is locked by an action of an electromagnetic clutch C₃ (see Fig.2) of the second parallel link assembly 3.

The end of the second parallel link assembly 3 of the retaining link mechanism 4 is provided with a horizontally moveable counterweight W₄ which can adjust a weight balance with the microscope W₁ in horizontal directions. Therefore, the horizontal movement of the counterweight W₄ and the vertical movement of the retaining link mechanism 4 relative to the pivot S are combined to allow the weight balance relative to the pivot S of the microscope W₁ to be completely adjusted. However, the balancing structure of the whole stand apparatus A relative to the pivot S is not a subject matter of the present invention, and the stand apparatus A may have any balance adjusting structure other than the one described above.

An L-shaped crank member 8 is pivotally supported at origin of the jib arm 5. One end of the crank member 8 and an upper end of the vertical front arm 6 are connected via a horizontal sub-arm 9, and the other end of the crank member 8 and a part of the mount 1 are connected via a vertical sub-arm 10. Therefore, if the retaining link mechanism 4 and the jib arm 5 are moved in the directions θ ₁ and θ ₂ respectively, the front arm 6 keeps vertical, namely does not tilt.

The lower end 7 of the front arm 6 is rotatable about a vertical axis α ₂ and can be optionally locked at a rotational position by an incorporated electromagnetic clutch C₄ (see Fig. 2). The lower end 7 is provided with a slider 11 which is substantially U-shaped in cross section and an arcuately curved lever 12 is slidably engaged with the slider 11. The slider 11 is provided with a dial 13 which can slide the curved lever 12 when turned.

To one end of the curved lever 12, a holder frame 14 is rotatably mounted about an axis α ₃. The holder frame 14 is provided at the proximal end thereof with an electromagnetic clutch C₅ which can lock the holder frame 14.

In order to adjust balance around the microscope W₁, a distal end 15 of the holder frame 14 is swingable about the proximal end of the holder frame 14 both rightward and leftward. The distal end 15 of the holder frame 14 extends to essentially direct under the front arm 6 and slanted as a whole. The distal end 15 of the holder frame 14 bifurcates and has a fork-like shape. The microscope W₁ is held between the bifurcated distal ends 15.

The microscope W₁ is held in a manner that observational directions are along the holder frame 14 relative to the distal end 15 of the holder frame 14. That is to say, if the longitudinal directions of the holder frame 14 in a planar view are assumed forward and rearward (shown as Y directions), the microscope W₁ is mounted in a state along the forward and rearward directions. The microscope W₁ is provided with a main ocular 16 through which a doctor observes, and an opposed scope 17 is provided on the opposite side of the ocular 16 so that an assistant doctor can observe. Thus, the observational direction of the main ocular 16 as an observational direction of the microscope W₁ will naturally conform to the forward and rearward directions.

Directions orthogonal to the forward and rearward directions are rightward and leftward directions (shown as X directions), and the distal ends 15 of the holder frame 14 are each positioned at the right and left sides of the microscope W₁. A side scope W₂ and video camera W₃, both are "auxiliary devices" to the microscope W₁, are mounted to the distal ends 15 of the holder frame 14. More specifically, at the distal ends 15 of the holder frame 14, an optical output 18 of the microscope W₁ is mounted in a rightwardly and leftwardly projecting manner. Since the side scope W₂ and video camera W₃, as auxiliary devices are mounted to the distal ends 15 which are positioned at the right and left sides relative to the microscope W₁, balancing change caused by absence or presence of the auxiliary devices is limited only to rightward and leftward, thereby being advantageous to weight balancing adjustment. The details of the balancing adjustment will be given later.

The optical output 18 is also a member about which the microscope W₁ rotates relative to the holder frame 14. The optical output 18 and an electromagnetic clutch C₆ adjacent thereto (see Fig. 1) are connected via a not-shown gear mechanism, and a positions of rotation about the optical output 18 are locked by the electromagnetic clutch C₆ at any optional positions.

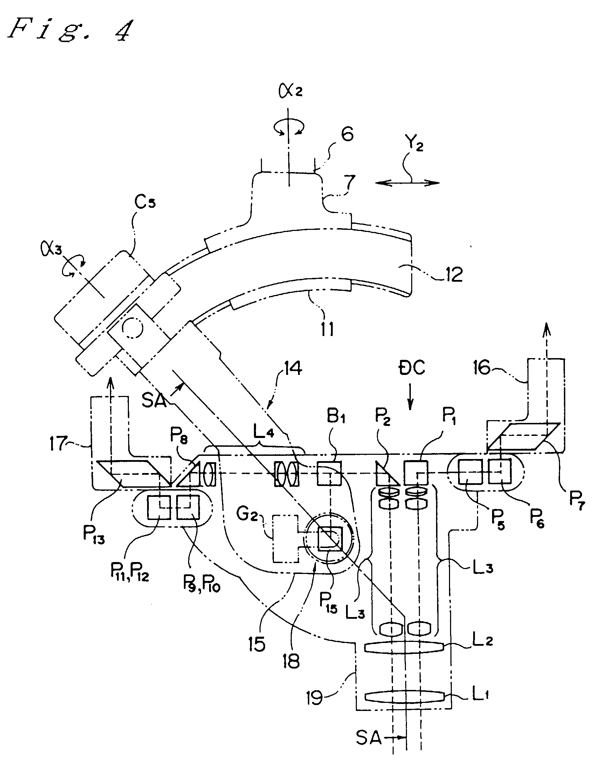

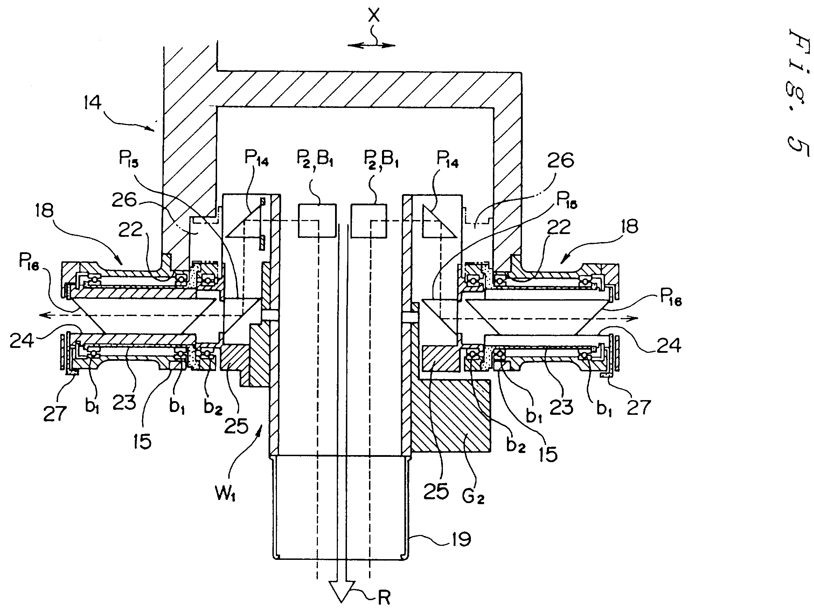

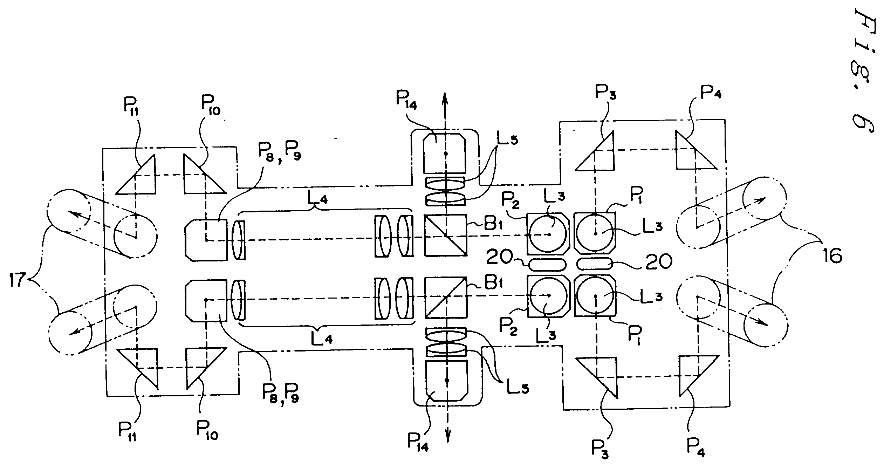

Next, the optical mechanisms in the microscope W₁ and in the optical output 18 are being described. The microscope W₁ is provided with a lens tube 19 which contains objective lenses L₁ and L₂ (see Fig. 4. Note that they are not shown in Fig. 5.). Four sets of zoom lens systems L₃ are vertically arranged above the objective lenses L₁ and L₂, and provided above the two of the zoom lens systems L₃ closer to the ocular 16 are a prism P₁ which reflects light flux to the external sides both rightward and leftward. Further provided above the other two of the zoom lens systems L₃ closer to the opposed scope 17 are a prism P₂ which reflects light flux to the side of the opposed scope 17. Out of the four zoom lens systems L₃, the pairs arranged in right-and-left relationship have two illumination introducers 20 (see Fig. 6) formed therebetween. Illuminating light R can be introduced through the illumination introducer 20 to the objective lens L₁ (see Fig. 5) immediately below.

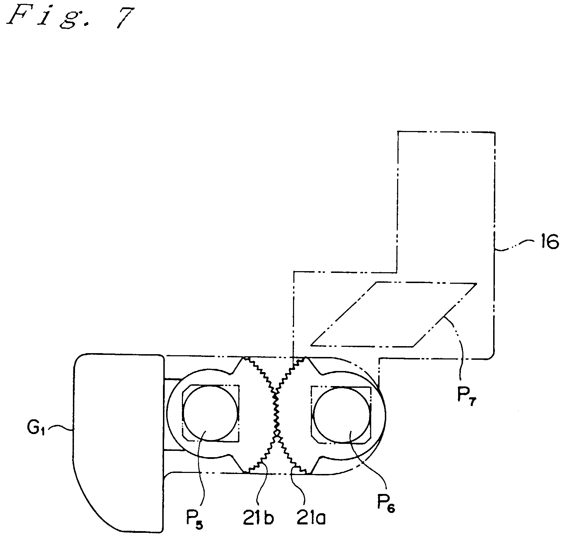

The light flux reflected from the prism P₁ on the side closer to the ocular 16 is introduced to the ocular 16 via prisms P₃ through P₇ As Fig. 7 shows, the ocular 16 is engaged with a counterweight G₁ via a pair of gears 21a, 21b. The pair of gears 21a, 21b engage with each other with very low resistance, so that rotation of the gears 21a, 21b for changing the angle of the ocular 16 is subject to extremely low resistance perceivable between the gears 21a, 21b. Therefore, angular movement of the ocular 16 does not cause any slight movement of the microscope W₁, thereby the views through the microscope W₁ do not blur. Although the gears 21a, 21b engage with each other so lightly, the ocular 16 is securely stopped at a position to which the ocular 16 is moved because the counterweight G₁ rotates to a direction where the weight of the ocular 16 is countervailed.

The light flux reflected from the prism P₂ on the side closer to the opposed scope 17 is introduced to the opposed scope 17 via a beam splitter B₁, Lens L₄, and prisms P₈ to P₁₃.

A part of the light flux directed to the opposed scope 17 is reflected by the beam splitter B₁ to the external sides both rightward and leftward. The reflected light flux is directed to the optical output 18 via a lens L₅ (not shown in Fig. 5), prisms P₁₄, P₁₅, and an image rotator P₁₆.

Next, the structure of the optical output 18 is being described. The optical outputs 18 project from circular holes 22 formed at the right and left distal ends 15 of the holder frame 14, and have a structure basically identical to each other between the right and left optical outputs 18. A cylindrical bush 23 is rotatably provided in the optical output 18 via a bearing b₁, and an inner cylinder 24 is provided in the cylindrical bush 23. Predetermined resistance provided between the cylindrical bush 23 and the inner cylinder 24 normally allows the bush 23 and the cylinder 24 to rotate integrally.

Another rotational element 25 is rotatably supported at the end of the inner cylinder 24 closer to the microscope W₁ via a bearing b₂. At the center of the rotational element 25 is fixed the prism P₁₅ which is positioned at the entrance ofthe optical output 18 and rotates together with the rotational element 25.

Both the bush 23 and the rotational element 25 have at a portion thereof a gear part which is engaged with a gear 26 provided at the right and left distal ends 15 of the holder frame 14. Gear ratio of each engaging part relative to the gear 26 allows the cylindrical bush 23 and the rotational element 25 to rotate just half of the rotational angle of the microscope W₁.

An operating lever 27 is fixed at the end of the inner cylinder 24, and rotating the lever 27 manually allows only the inner cylinder 24 (image rotator P₁₆) to rotate against the predetermined resistance, relative to the cylindrical bush 23 which is engaged with the gear 26.

On the right side of the microscope W₁, a counterweight G₂ is provided so as to rotate concentrically with the pivot point of the microscope W₁. The counterweight G₂ and the microscope W₁ are provided therebetween with predetermined frictional force to maintain a state after the counterweight G₂ is manually rotated. The counterweight G₂ is rotated in the counter direction to that the microscope W₁ is rotated, so as to balance when the microscope W₁ is rotated about the optical outputs 18.

Some advantages of the embodiments are being enumerated below.

### Inhibiting the Center of Gravity of the Microscope in Rotation from Fluctuating:

The side scope W₂ and video camera W₃, as auxiliary devices to the microscope W₁ and mounted at the distal ends 15 of the holder frame 14 allow only the microscope W₁ to be moved, namely the auxiliary devices are immobilized, when the microscope W₁ is rotated about the optical output 18. Thus, the rotated microscope W₁ does not affect the good whole balance kept by the stand apparatus.

### Easy Optical Connectability of the Auxiliary Devices:

The microscope W₁ rotatably supported at the distal end 15 of the holder frame 14 about the optical output 18 defines at the pivot center thereof a passage for light flux and allows an optical connection between the microscope W₁ and the auxiliary devices easily.

### Half Rotation Mechanism relative to the Rotation of the Microscope:

Since the prism P₁₅ positioned at the entrance of the optical output 18 and the image rotator P₁₆ positioned within the optical output 18 rotate by half the rotated angle of the microscope W₁, the images in the side scope W₂ and video camera W₃ and available through the optical output 18 do not rotate even if the microscope W₁ is rotated.

### Optional Rotation Mechanism of the Image Available through the Optical Output

Manual rotation of the image rotator P₁₆ allows an image available through the optical output 18 to be optionally rotated, so that it is advantageous or convenient that an image thorough the side scope W₂ or video camera W₃ can be changed to any desired orientations.

### Vertical Illumination Effect

The two illumination introducers 20 respectively formed between the four zoom lens systems L₃ in the right and left directions allow illuminating light R through the introducers 20 to be illuminated from directly above to an area to be observed in magnification, so that the illuminating light can securely reach to the bottom of an affected part even if the affected part is small and deep, and bright and clear image can be observed.

In the aforementioned embodiments, the holder frame 14 is shaped to have distal ends 15 which are bifurcated at the middle of the frame 14, and further the distal ends 15 can be bifurcated at the origin of the frame 14.

Although the stand apparatus A according to the embodiment has a mount 1 which is to be installed on a floor of an operating room, the mount can be of a type which is to be mounted either on a ceiling or on a side wall of an operating room. Such types of stand apparatuses which are provided with a mount to be installed other than on a floor have an advantage that allows the floor to be more effectively used.

## Claims

1. A stand apparatus (A) for a microsurgery aid, the stand apparatus (A) including a holder frame (14) moveable to any optional spatial position and being adapted to support a microscope (W₁) and one or more auxiliary devices (W₂,W₃) at the distal end (15), characterised in that the distal end (15) of said holder frame (14) bifurcates to have a fork-like shape between which bifurcated distal end a microscope (W₁) is supported in use, at least one side of the bifurcated distal end (15) being adapted to support an auxiliary device.

2. A stand apparatus according to claim 1, wherein the distal end (15) of said holder frame (14) includes at least one optical output (18) to which a microscope can be rotatably supported, the auxiliary device being optically connected in use to the optical output (18).

3. A stand apparatus according to claim 2, further comprising reflection means for passing light from a microscope mounted to the stand into at least one optical output (18), the reflection means including a prism (P₁₅) or mirror which is coupled to a microscope, in use, so as to rotate relative to the optical output (18) by half the rotated angle of the microscope (W₁).

4. A stand according to claim 2 or claim 3, further comprising an image rotator (P₁₆) provided within the optical output (18) and being rotatable relative to the optical output by half the rotated angle of the microscope (W₁).

5. A stand according to claim 4, wherein the image rotator (P₁₆) can be manually rotated.

6. A microsurgery aid comprising a stand apparatus (A) according to any of the preceding claims; a microscope (W₁) mounted to the distal end (15) of the holder frame (14); and at least one auxiliary device (W₂,W₃) mounted to a respective side of the distal end.

7. A microsurgery aid according to claim 6, wherein the auxiliary device is a side scope (W₂) or video camera (W₃).
